# EUROPEAN PATENT APPLICATION

(11) **EP 1 932 530 A1**
(43) Date of publication of application: **18.06.2008**
(21) Application number: 06782270.0
(22) Date of filing: 03.08.2006
(51) Int. Cl.: A61K 31/7016, A23G 9/00, A23L 1/30, A23L 2/52, A61K 8/60, A61K 31/122, A61K 31/375, A61K 31/7008, A61K 31/702, A61K 31/7048, A61K 31/7076, A61K 31/726, A61P 1/02, A61P 17/16, A61P 43/00, A61Q 11/00, A61Q 19/00, C07H 3/04, C07H 3/06

(54) **AGENT FOR ENHANCING COLLAGEN PRODUCTION AND UTILIZATION OF THE SAME**

(30) Priority: 11.08.2005 JP 2005232679
(71) Applicant: KABUSHIKI KAISHA HAYASHIBARA SEIBUTSU KAGAKU KENKYUJO, Okayama-shi, Okayama 700-0907 (JP)
(72) Inventor: MIYATA, Satomi, Okayama-shi Okayama 7000907 (JP); USHIO, Shimpei, Okayama-shi Okayama 7000907 (JP); IWAKI, Kanso, Okayama-shi Okayama 7000907 (JP); MIYAKE, Toshio, Okayama-shi Okayama 7000907 (JP)
(74) Representative: Daniels, Jeffrey Nicholas
(86) International application number: PCT/JP2006/315410
(87) International publication number: WO 2007/018124

(57) **Abstract**

The present invention has an object to provide a means for durably exerting an action of enhancing collagen-production, and the object is attained by providing a collagen-production enhancer which contains, as an effective ingredient, α,α-trehalose and/or a saccharide derivative thereof, and by a composition incorporated with the collagen-production enhancer to be used for such purpose.

## Description

### Technical Field

The present invention relates to a novel collagen-production enhancer and a composition for enhancing collagen-production, particularly, to a collagen-production enhancer containing α,α-trehalose and/or a saccharide derivative thereof and to a composition for enhancing collagen-production, which contains the collagen-production enhancer.

### Background Art

After coming into this aging society, age-related reduction of skin thickness and metabolism in the skin are troublesome for most of the middle and senior generations, particularly, for women. Representative examples of such are the changes in body conditions such as facial changes accompanied by distinctively noticeable facial wrinkles/finely-wrinkles, facial skin sagging, loss of skin fitness, and reduction of skin elasticity. Cosmetics, which are supplemented with collagen and mucopolysaccharides such as hyaluronic acid for maintaining humectancy in the skin, have been explored so far to prevent skin aging, but they could not have succeeded in a sufficient effect on such purpose. Recently, progressed researches on aging have revealed that a remarkable reduction of the level of collagen fibers as a constituent for the dermis is a major causative of skin aging. It has been also indicated that the reduction of collagen fibers correlates with facial changes such as induction of facial wrinkles/finely-wrinkles, loss of skin fitness, induction of facial skin sagging, and reduction of skin elasticity. Although there exist many causatives of skin aging, it finally ends up the reduction of collagen metabolism as a result of the reduction of collagen-producing ability of fibroblasts in the dermis or the reduction of proliferation ability of fibroblasts *per se.*

The reduction of collagen production in fibroblasts not only accelerates skin aging but also lowers biodefence ability as a major function of the skin and also weakens tissues and organs in living bodies, e.g., bone and blood vessels, whose structures are maintained by collagen, and it may affect healthy conditions of living bodies. Since collagen, as a protein, is not substantially absorbed by living bodies smoothly if only taken orally or applied externally to the skin and it could not augment the collagen-production activity of fibroblasts, collagen per se could not be a primary preventive/therapeutic material for skin aging. To prevent the aging of tissues including the skin, blood vessels and bone and to maintain/promote the health of living bodies, it is desirable to explore agents for enhancing collagen-production that are safe and capable of continuously augmenting the production level of collagen as a major constituent of tissues and organs including the dermis, bone, and blood vessels to a desired healthy level; there has been proposed a collagen-production enhancer containing a derivative of L-ascorbic acid and a royal jelly (Japanese Patent Kokai No. 2003-171290) and it has been also reported that saccharides such as glucose enhance collagen production (see, for example, Han D. C., Journal of the American Society of Nephrology, Vol. 10, No. 9, pp. 1,891-1,899 (1999). These components, however, may have problems in stability depending on their formulations and conditions in use and therefore a development of novel, improved collagen-production enhancer has been desired.

α,α-Trehalose is a disaccharide, where two glucose molecules are linked together via their reducing groups, and saccharide derivatives thereof are those which have an α,α-trehalose structure within their molecules and consist of at least three glucose molecules; and these saccharides have been being increased in demand, as substituents for sucrose, for food products and external dermal agents, which use their properties of moisturizing action, preventing retrogradation of starch, inhibiting deterioration of lipids, and preventing degeneration of proteins, in the fields of foods, cosmetics, quasi-drugs, and pharmaceuticals.

α,α-Trehalose and saccharide derivatives thereof are conventionally known compounds and many patent applications relating to their production methods and uses have been applied for by the same applicant as the present invention (see, for example, Japanese Patent Kokai Nos. 143876/95, 213,283/95, 2000-228980, and International Patent Publication No. WO 2004/056216). α,α-Trehalose has been disclosed for use as a filler for a collagen-production enhancer (see, for example, Japanese Patent Kokai No. 2003-171290), however, any of the above-identified publications neither disclose nor suggest the fact that α,α-trehalose and/or saccharide derivatives thereof exert a distinct enhancing action on the collagen production by L-ascorbic acid or the like (designated as "L-ascorbic acids", hereinafter), and there has been no report that focused on the collagen-production enhancement by α,α-trehalose and/or saccharide derivatives thereof.

### Disclosure of Invention

The present invention has an object to provide a means for effectively enhancing the collagen production by L-ascorbic acids.

The present inventors energetically made continued researches to solve the above object and unexpectedly found that α,α-trehalose and/or saccharide derivatives thereof quite effectively enhance the collagen production by L-ascorbic acids, and thus they accomplished this invention.

The present invention was made based on the finding that α,α-trehalose and/or saccharide derivatives thereof quite effectively enhance the collagen production by L-ascorbic acids in humans and animals. These α,α-trehalose and/or saccharide derivatives thereof are stable and safe and they can be applied easily and comfortably to humans and animals for a relatively long-term successive use to prevent skin aging, maintain and promote beauty and health conditions, and to strengthen bone and blood vessels, etc., without a fear of causing serious side effect.

### Best Mode for Carrying Out the Invention

The α,α-trehalose and/or saccharide derivatives thereof used in the present invention exert an action of enhancing the collagen production by L-ascorbic acids in humans and animals and any of these saccharides can be advantageously used in the present invention. In the collagen-production enhancer of the present invention, any of which is acceptable as long as it contains one or more of these α,α-trehalose and/or saccharide derivatives thereof in an effective amount in total.

The α,α-trehalose and/or saccharide derivatives thereof used in the present invention can be prepared by various methods such as fermentation, enzymatic method, and synthetic method, and those which contain such saccharides in the form of a syrup, massecuite, amorphous powder, crystalline powder of non-centrifugal sugar, or crystal can be arbitrarily used. The α,α-trehalose and/or saccharide derivatives thereof should not necessarily be highly purified ones and any of those which are in the form a composition unseparated from other saccharides coexisted during their preparations, as well as those in a partially or highly purified form, those which contain sugar alcohols prepared by hydrogenating reducing-saccharides formed and coexisted during their preparations, and those in a mixture form with other adequate components which do not substantially hinder the action of collagen-production enhancement by L-ascorbic acids when used in humans and animals. Since the present invention does not relate to a preparation of α,α-trehalose and saccharide derivatives thereof, it does not mention such a preparation in detail, however, when economical aspects are valued, preferable methods are, for example, those disclosed in Japanese Patent Kokai Nos. 143876/95, 213283/95, and 2000-228980, and International Patent Publication No. WO 2004/056216, where partial starch hydrolyzates are subjected to the action of a non-reducing saccharide-forming enzyme and a trehalose-releasing enzyme. These methods yield α,α-trehalose and saccharides containing the same from starch as a low cost material in a relatively high yield. Examples of commercialized α,α-trehalose include "TREHA", a product name of a food grade α,α-trehalose powder containing, on a dry solid basis (d.s.b.), at least 98% by weight of hydrous crystalline trehalose, commercialized by Hayashibara Shoji Inc., Okayama, Japan. Examples of saccharides containing saccharide derivatives of α,α-trehalose include "HALLODEX", a product name of a syrup containing at least 50% by weight, d.s.b., of α-maltosyl α,α-trehalose and about 7% by weight, d.s.b., of other saccharide derivatives of α,α-trehalose, commercialized by Hayashibara Shoji Inc., Okayama, Japan; and "TORNARE", a product name of a syrup containing at least 50% by weight, d.s.b., of α-maltosyl α,α-trehalose and about 10% by weight, d.s.b., of saccharide derivatives of α,α-trehalose, commercialized by Hayashibara Biochemical Laboratories Inc., Okayama, Japan, which are prepared by hydrogenating "HALLODEX" to covert the coexisting reducing sugars to their corresponding sugar alcohols.

The saccharide derivatives of a,a-trehalose used in the present invention should not specifically be restricted to specific ones as long as they enhance the collagen production by L-ascorbic acids. Concretely, any one or more saccharides selected from the group consisting of non-reducing oligosaccharides, composed of at least three glucoses and having a trehalose structure intramolecularly, can be used. More concretely, the saccharide derivatives of α,α-trehalose mean those which any of mono-, di-, tri-, and tetra-glucoses is/are bound to either or both of the glucose residues of α,α-trehalose molecule.

Among the above-identified saccharide derivatives of α,α-trehalose, the following can be advantageously used in the present invention; α-glucosyl α,α-trehalose, a-maltosyl a,a-trehalose, α-maltotriosyl α,α-trehalose, α-maltotetraosyl α,α-trehalose, etc., which have a trehalose structure as an end unit at their molecular termini and have a relatively strong action of enhancing the collagen production by L-ascorbic acids. Particularly, preferable saccharides are those which contain α-maltosyl α,α-trehalose as a main component and one or more other saccharides selected from α-glucosyl α,α-trehalose, α-maltotriosyl α,α-trehalose, and other α-glycosyl α-glucose, disclosed in, for example, International Patent Publication No. WO 2004/056216. In this instance, preferable saccharides are those which contain at least about 5% by weight, d.s.b., preferably, at least about 10% by weight, d.s.b., and more preferably, at least about 30% by weight, d.s.b., of α-maltosyl α,α-trehalose to the total amount of saccharides.

To the collagen-production enhancer of the present invention can be added one or more of the following ingredients in an amount that does not hinder the action of enhancing collagen production by the collagen-production enhancer; saccharides such as glucose, fructose, glucosamine, lactose, sucrose, α,β-trehalose, β,β-trehalose, lactosucrose, maltooligosaccharides, and syrups of starch hydrolyzates; cyclic saccharides such as cyclodextrins and a cyclotetrasaccharide having the structure of cyclo{→6)-α-D-glucopyranosyl-(1→3)-α-D-glucopyranosyl-(1→6)-α-D-glucopyranosyl-(1→3)-α-D-glucopyranosyl-(1→} disclosed in International Patent Publication No. WO 02/10361 applied for by the same applicant as the present invention, and a cyclotetrasaccharide having a structure of cyclo{→6)-α-D-glucopyranosyl-(1→4)-α-D-glucopyranosyl-(1→6)-α-D-glucopyranosyl-(1→4)-α-D-glucopyranosyl-(1→} disclosed in Japanese Patent Kokai No. 2005-95148 (Japanese Patent Application No. 2004-174880); sugar alcohols such as erythritol, mannitol, sorbitol, xylitol, maltitol, and hydrogenated starch syrups; high-sweetened sweeteners such as L-aspartyl-L-phenylalanine methyl ester (aspartame), stevia extract, sucralose, and acesulfame K; mucopolysaccharides such as pullulan, carrageenan, and chondroitin sulfate, and salts thereof; natural gums; chitin; chitosan; synthetic high polymers such as carboxymethyl cellulose; collagen; and thickeners such as gelatin.

Into the collagen-production enhancer of the present invention can be optionally incorporated the following ingredients other than α,α-trehalose and saccharide derivatives thereof; ingredients with an action of enhancing collagen production, such as an extract from buds of trees of *Fagus crenata* disclosed in, for example, Japanese Patent Kokai No. 203952/98. If necessary, the collagen-production enhancer can be used in combination with one or more ingredients from deep sea water; minerals such as calcium of oyster shell; emulsifiers; flavors; spices; colors; vitamins such as vitamin B₁, vitamin B₂, vitamin B₆, vitamin B₁₂, L-ascorbic acid, vitamin E, vitamin P or the like such as rutin, hesperidin, naringin, and derivatives of these vitamins; amino acids; coenzyme Q10 (CoQ10); and adenosine and their derivatives and salts such as those in the form of a mono-, di-, or tri-phosphate. Among which, mucopolysaccharides such as chondroitin sulfate, and adenosine and their derivatives and salts such as those in the form of a mono-, di-, or tri-phosphate can further increase the action of enhancing collagen-production by α,α-trehalose and/or saccharide derivatives thereof when used in combination with the collagen-production enhancer of the present invention. Usually, the selection standard for the above-identified ingredients is appropriately chosen depending on the necessity in the fields where the collagen-production enhancer of the present invention is used.

The term "L-ascorbic acid or the like" (L-ascorbic acids) as referred to as in the present invention means L-ascorbic acid and derivatives thereof, which are capable of allowing fibroblasts to induce collagen production when used intact or after decomposed or modified by enzymes, etc. Concretely, there can be illustrated L-ascorbic acid, L-ascorbic acid glycosides such as L-ascorbic acid 2-glucoside, acyl derivatives of L-ascorbic acid glycosides, phosphates and sulfates of L-ascorbic acid, and salts thereof. In general, L-ascorbic acid is present in living bodies of humans and animals in an amount sufficient for collagen production by fibroblasts and it should not be needed to supplement L-ascorbic acids to the collagen-production enhancer of the present invention, however, L-ascorbic acids should preferably be used in combination when the collagen-production level in the living bodies is low due to the lack of L-ascorbic acid. In this case, the standard dose of L-ascorbic acids requisite for the collagen production in humans and animals can be appropriately selected depending on respective fields where the collagen-production enhancer of the present invention is used.

Explaining the use of the collagen-production enhancer of the present invention with reference to the case for use in humans, the enhancer exerts the action of collagen production independently of peroral or parenteral administration routes. With the above-mentioned action, the collagen-production enhancer effectively enhances collagen production independently of its peroral and parenteral use. Varying depending on use, the collagen-production enhancer of the present invention can be usually taken perorally or intubationally (e.g., through gastric and intestinal administration routes) in the form of a food, liquid, syrup, powder, granule, tablet or capsule, and it can be also administered percutaneously in the form of an external dermal agent. In other cases, the enhancer can be advantageously used parenterally to enhance collagen production in the desired parts, strengthen tissues and organs, and promote healing of affected parts of injury, burn wound, and inflammation in the form of an ophthalmic solution; injection; infusion; peritoneal or pleural perfusal; solution for washing or disinfecting organs, injured parts, eyes, nasal cavities, burn wounds, and inflammatory parts; peritoneal dialysate; and preservation solution for tissues and organs. The term "external dermal agent" as referred to as in the present invention means any of which contains α,α-trehalose and/or saccharide derivatives thereof in a composition form and directly contacts with the skin, such as those in the form of a cosmetic, quasi-drug, and pharmaceutical, as well as everyday sundries such as detergents.

Independently of peroral, percutaneous, and injection (including intraperitoneal perfusion or the like) administration routes, the effective ingredient in the collagen-production enhancer of the present invention is promptly absorbed by living bodies to enhance the collagen production by L-ascorbic acids in fibroblasts existing in the dermis, tissues, bone, organs, etc., and it stably enhances collagen production in humans and animals when ingested by them, followed by recovering a lowered collagen-production-ability of the skin induced by senescence accompanied by aging, dialysis, tissue damage induced by surgeries and diseases accompanied by inflammation, and or damages by factors such as ultraviolet rays; imparting fitness and moistened property to the skin; removing wrinkles/finely-wrinkles; recovering skin firmness, and strengthening tissues and organs including bone and blood vessels; and curing wounds. Particularly, in the case of administering percutaneously, α,α-trehalose and/or saccharide derivatives thereof continuously enhance the collagen production by L-ascorbic acids, strengthen both skin dermis and cornified layer to improve biophylaxis, and prevent/improve skin roughness. Thus, such administration smoothly recovers a reduced collagen-production-ability of the skin induced by senescence accompanied by aging or of skin damaged by factors such as ultraviolet rays and harmful microorganisms, imparts fitness and moistened property to the skin, removes wrinkles/finely-wrinkles, improves skin roughness, and recovers skin firmness. When used as a cosmetic to be applied directly to the skin including scalp, the collagen-production enhancer of the present invention improves the prophylactic and therapeutic effects on skin diseases through the above-mentioned effects and has effects on hair regeneration, growth, etc. In the case of directly applying the collagen-production enhancer of the present invention and/or compositions containing the same as a cosmetic or the like, they can be accelerated to penetrate into the skin by using, for example, an apparatus for iontophoresis disclosed in International Patent Publication No. WO 01/60388 applied for by the same applicant as the present invention. Thus, the collagen-production enhancer of the present invention can be also used as an agent for inhibiting the formation of or improving wrinkles/finely-wrinkles, agent for hair regeneration or growth, or agent for strengthening tissues. The collagen-production enhancer can be also easily used for humans and animals to maintain and promote their health conditions in the form of a tonic, health food, supplemental health food, food for special dietary uses, health-promoting food, cosmetic, quasi-drug, pharmaceutical, feed, baits for fish, pet food, other daily goods, etc.

Varying depending on the kind, age, sex, etc., of humans and animals including pet animals to be administered, the intake amount or the dose of the collagen-production enhancer of the present invention is usually from 0.001 g to 2 g, preferably, from 0.01 g to 1 g per kg body weight of a subject in terms of a dry weight of α,α-trehalose and/or saccharide derivatives thereof at a frequency of once or several times a day and, depending on its efficacy, every successive days or at an interval of one or more days, when taken orally or intubationally or administered parenterally except for percutaneous administration. The collagen-production enhancer as a parenterally administrable tonic, health food, supplemental health food, food for special dietary uses, health-promoting food, quasi-drug, pharmaceutical, feed, baits for fish, pet food, etc., can be used in the form of a liquid, tablet, powder, granule, paste, syrup, capsule, etc., depending on use. In such cases, the above products usually contain at least 0.1% by weight, d.s.b., preferably, at least 1% by weight, d.s.b., of α,α-trehalose and/or saccharide derivatives thereof. When used as an external dermal agent to be directly contacted with skin, the collagen-production enhancer of the present invention contains 0.1 to 20% by weight, d.s.b., preferably, 1 to 20% by weight, d.s.b., and more particularly, to attain a stable effect, 2 to 20% by weight, d.s.b., of α,α-trehalose and/or saccharide derivatives thereof to the total amount of the agent, and it can be allowed to be contacted with skin by spraying and applying to skin at a frequency of one to several times a day and, depending on its effect, every successive days or at an interval of one or more days. The upper limit is made "20% by weight" due to the fact that no effect might not only be expected but also it may cause problems in formulation in terms of physical property and feeling of use, depending on the form of an external dermal agent to be used. It goes without saying that there is no upper limit of α,α-trehalose and/or saccharide derivatives thereof to be added to such an external dermal agent when it does not have the above problems of undesirable physical property and feeling of use. In the case that an external dermal agent incorporated with a saccharide derivative of α,α-trehalose has a sticky feeling of use, it can be used in combination with thickeners such as sodium polyacrylate, sodium polyglutamate, scleroglucan, quinseed extract, and water-soluble polysaccharides such as pullulan; ethanol; and a mixture of butylene glycol and glycerol in a weight ratio of 1:20 to 20:1. As described above, the collagen-production enhancer of the present invention is useful in itself and it can be arbitrarily used in the form of a composition with other ingredients. Such a composition containing the collagen-production enhancer of the present invention is prepared by appropriately employing the following steps according to appropriate compositions selected depending on the kinds of animals to be applied and methods for intake and administration; steps of mixing α,α-trehalose and/or saccharide derivatives thereof with one or more ingredients usable in any of the fields of the above-mentioned foods, cosmetics, quasi-drugs, pharmaceuticals, feeds, baits for fish, or pet foods, based on their respective contents suitable for final uses, into a desired composition containing α,α-trehalose and/or saccharide derivatives thereof; and optionally shaping the composition to have a desired shape. The collagen-production enhancer of the present invention and a composition containing the same can be prepared by appropriately using one or more methods of diluting, concentrating, drying, filtrating centrifuging, mixing, kneading, dissolving, melting, spreading, suspending, emulsifying, soaking, penetrating, dispersing, applying, coating, spraying, injecting, crystallizing, solidifying, reverse micelle technique, etc. Preparations such as the aforementioned pharmaceuticals to be administered to living bodies, whose administration methods are different from peroral/intestinal administration and dermal application, should preferably be removed microorganisms and pyrogens therefrom before use.

The ingredients, which are acceptable for use in peroral, percutaneous, or external application to humans and animals, used in the present invention include the above-identified ones or others generally used in respective fields where the compositions according to the present invention are used; water, alcohols, amylaceous substances, proteins, amino acids, fibers, saccharides, lipids, fatty acids, vitamins, minerals, flavors, colors, sweeteners, seasonings, spices, antiseptics, antioxidants, emulsifiers, surfactants, powders, etc.

With an indication of noticing a collagen-production-enhancing-ability, the collagen-production enhancer of the present invention and compositions containing the same can be used for such purpose. The form of the collagen-production enhancer and compositions containing the same should not specifically be restricted. Preferable forms of external dermal agents are, for example, those disclosed in International Patent Publication No. WO 2004/056216, such as basic cosmetics including those in the form of a lotion, milky lotion, cream, solid, solid powder, jelly, moose, pack, face mask, film, etc.; face-wash cosmetics, bath cosmetics, oral cosmetics, sun-burn/sun-tan cosmetics, make-up cosmetics, hair cosmetics (including restorers and agents for hair growth), and daily goods such as kitchen detergents that directly affect the skin. Examples of the pharmaceuticals with the collagen-production enhancer of the present invention include injections for intracutaneous, subcutaneous, and intravascular administrations; intraperitoneal perfusates and washes; dialysates for peritoneal dialysis; solutions for washing and disinfecting wounds; ophthalmics; nebulas; external preparations, etc. Preferred examples of the foods with the collagen-production enhancer of the present invention are frozen desserts such as an ice cream, ice candy, and sherbet; syrups such as *"korimitsu"* (a sugar syrup for shaved ice); spreads and pastes such as a butter cream, custard cream, flour paste, peanut paste, and fruit paste; Western cakes such as a chocolate, jelly, candy, gummy jelly, caramel, chewing gum, pudding, cream puff, and baked confectionery including sponge cake; processed fruits and vegetables such as a jam, marmalade, "*syrup-zuke*" (a fruit pickle), and "*toka*" (a conserve); Japanese cakes such as "*manju*" (a bun with a bean-jam), "*uiro*" (a sweet rice jelly), *"an"* (a bean jam), "*yokan*" (a sweet jelly of beans), "*mizu-yokan*" (a soft adzuki-bean jelly), *pao de Castella,* "amedama" (a Japanese toffee), and rice cake; noodles; "*gyuhi*" (a starch paste); cocked rice; daily dishes; dried foods; retort foods; frozen foods; and seasonings such as a soy sauce, powdered soy cause, *miso,* powdered miso, mayonnaise, dressing, vinegar, "*sanbai-zu*" (a sauce of sugar, soy sauce and vinegar), sweet sake, table sugar, and coffee sugar. Preferable examples of the beverages with the collagen-production enhancer include alcohols such as a synthetic sake, fermented liquor, sparkling wine, wine, and spirit; and soft drinks such as a juice, mineral beverage, carbonated beverage, sour milk beverage, lactic fermenting beverage, isotonic drink, nutritious supplement drink, teas including green tea, tea, and oolong tea, as well as other beverages including coffe and cocoa. The above-identified compositions according to the present invention usually contain 0.01 to 20% by weight, preferably, 0.1 to 10% by weight of the collagen-production enhancer of the present invention to the total weight of the compositions each.

The present invention is explained in more detail with reference to the following Experiments.

### <Experiment 1: Influence of α,α-trehalose on the collagen production by L-ascorbic acids>

Using "fibrocell NHDF", a normal human dermal fibroblast cell, commercialized by Kurashiki Spinning Co., Ltd., Okayama, Japan (abbreviated as "NHDF cells", hereinafter), experiment for studying the influence of α,α-trehalose on the collagen production by L-ascorbic acids was conducted based on the method described in Bioscience, Biotechnology, and Biochemistry, Vol. 68, No. 4, pp. 767-773, 2004. The method is outlined below:

### <Test solutions>

Test solutions were prepared by dissolving L-ascorbic acid 2-glucoside, a reagent grade specimen commercialized by Hayashibara Biochemical Laboratories Inc., Okayama, Japan (may be abbreviated as "AA-2G", hereinafter), and α,α-trehalose, a reagent grade specimen commercialized by Hayashibara Biochemical Laboratories Inc., Okayama, Japan, in Dulbecco minimum essential medium containing 10% (v/v) fetal calf serum and an antibiotic (abbreviated as "D-MEM", hereinafter) to give a final concentration of 50 µM/ml of AA-2G and a final concentration of 7.5, 15 or 30 mg/ml of α,α-trehalose.

### <Method for quantifying collagen production in NHDF cells>

NHDF cells, which had been successively cultured in D-MEM medium, were collected, re-suspended in a fresh preparation of D-MEM medium, inoculated to 96-well microplates at a cell concentration of 2.5 x 10⁴ cells/200 µl/well, and cultured for 24 hours. Thereafter, the supernatants of the cultures were removed by sucking, and further cultured for five days after addition of any of the test solutions in a volume of 200 µl/well. As controls, the cells, which had been cultured for 24 hours, were further cultured for five days after addition of 200 µl/well of D-MEM medium, D-MEM medium supplemented with α,α-trehalose at a concentration of 30 mg/ml, or D-MEM medium supplemented with AA-2G at a concentration of 50 µM/ml. After completion of culturing, the supernatants of the cultures were removed by sucking, admixed with 50 µl/well of 1 M acetic acid solution containing 1 mg/ml of pepsin, and shaken for four hours to solubilize collagen adhered to the cell surfaces, followed by quantifying the content of collagen accumulated in the cells using "Sircol Collagen Quantification Kit", commercialized by Biocolor Ltd. These control and test solutions were respectively experimented with three wells, followed by calculating a mean value for collagen-production levels in the wells. All the cultures of the NHDF cells were conducted in a 5% (v/v) CO₂ incubator. The results are in Table 1 where the values are expressed with relative values in terms of the collagen-production level of NHDF cells, which had been cultured in D-MEM medium containing AA-2G, being regarded as 100.

**Table 1**

| | Culture | Concentration of α,α-trehalose (mg/ml) | Collagen-production level |
|---|---|---|---|
| Control | D-MEM medium | 0.0 30.0 | 43 41 |
| | D-MEM medium with 50 µM AA-2G | 0.0 | 100 |
| Test solution | D-MEM with | 7.5 | 150 |
| | 50 µM AA-2G | 15.0 | 217 |
| | | 30.0 | 264 |

As evident from Table 1, the collagen-production levels of NHDF cells, which had been cultured in D-MEM medium with no addition of AA-2G and the one with addition of α,α-trehalose, were respectively 43 and 41, showing that a sole use of α,α-trehalose gave no enhancing action of the collagen production by AA-2G. While the NHDF cells cultured in D-MEM media with 50 µM AA-2G and further supplemented with any of 7.5, 15 and 30 mg/ml of α,α-trehalose gave collagen production levels of 150, 217 and 264, respectively, meaning that the collagen production by AA-2G was enhanced by the addition of α,α-trehalose in a dose dependent manner.

### <Experiment 2: Influence of L-ascorbic acids on the collagen production>

Influence of α,α-trehalose and maltose on collagen production using NHDF cells was examined by the following experiment:

### <Test solutions>

Test solutions were prepared by dissolving either AA-2G; hydrous crystalline α,α-trehalose, a reagent grade specimen, commercialized by Hayashibara Biochemical Laboratories Inc., Okayama, Japan; or hydrous crystalline maltose, a reagent grade specimen, commercialized by Hayashibara Biochemical Laboratories Inc., Okayama; Japan, in D-MEM medium to give a final concentration of 50 µM of AA-2G and 0.6, 1.7, 5.0 or 15.0 mg/ml of α,α-trehalose or maltose.

### <Method for quantifying the collagen-production level in NHDF cells>

The methods for culturing NHDF cells and quantifying the collagen-production level of the cells were conducted similarly as in Experiment 1. The collagen level in each culture supernatant of NHDF cells was determined in such a manner of collecting the culture supernatant and quantifying the collagen released in the supernatant by using "Sircol Collagen Quantification Kit". As a control, D-MEM medium containing 50 µM AA-2G was used. These control and test solutions were respectively experimented using three wells, followed by calculating a mean value for collagen-production levels in the wells. The results are in Table 2 where the values are expressed with relative values for either or both of the levels of collagen, that had sedimented over the cell surfaces or released in the supernatants, in terms of their collagen-production levels of NHDF cells, which had been cultured in D-MEM medium containing 50 µM AA-2G, being regarded as 100.

**Table 2**

| Culture | | Concentration of α,α-trehalose or maltose (mg/ml) | Collagen-production level | | |
|---|---|---|---|---|---|
| | | | Sedimented over cell surface (mg/ml) | In supernatant | Total |
| Control | D-MEM containing 50 µM AA-2G | 0 | 100 | 100 | 100 |
| Test solution | | 0.2 | 101 | 103 | 101 |
| | D-MEM containing | 0.6 | 118 | 203 | 131 |
| | 50 µM AA-2G plus | 1.7 | 137 | 250 | 154 |
| | α,α-trehalose | 5.0 | 157 | 232 | 169 |
| | | 15.0 | 207 | 1058 | 335 |
| | | 0.2 | 98 | 101 | 99 |
| | D-MEM containing | 0.6 | 85 | 134 | 92 |
| | 50 µM AA-2G plus | 1.7 | 63 | 288 | 97 |
| | maltose | 5.0 | 25 | 256 | 61 |
| | | 15.0 | 11 | 228 | 44 |

As evident from Table 2, when cultured in D-MEM media containing 50 µM AA-2G and being supplemented with α,α-trehalose, enhancements of the collagen production by AA-2G were observed in both the levels of collagen sedimented over the cell surfaces and that released in the supernatants, in terms of the collagen-production levels of NHDF cells, which had been cultured in D-MEM medium supplemented with α,α-trehalose, in a dose dependent manner. While in the case of the cultures with maltose, it was confirmed that enhancements of collagen production in the culture supernatants were observed, however, the level of collagen sedimented over the cell surfaces and the total level of collagen, which had sedimented over cell surfaces and released in the supernatants, were inhibited as the increase of the additional amount of maltose.

### <Experiment 3: Influence of α,α-trehalose and saccharide derivatives thereof on the collagen production by L-ascorbic acids>

Based on the confirmation that α,α-trehalose has an action of enhancing the collagen production by L-ascorbic acid, it was experimented as follows to confirm whether saccharide derivatives of α,α-trehalose have such a similar action and effect.

### <Test solutions>

Test solutions were prepared by dissolving in D-MEM medium any one of AA-2G, hydrous crystalline α,α-trehalose, a reagent grade specimen with a purity of at least 98%, commercialized by Hayashibara Biochemical Laboratories Inc., Okayama, Japan; "HALLODEX", a product name of a syrup of saccharide derivatives of α,α-trehalose, having a solid content of about 75% and containing, on a dry solid basis, about 53% of α-maltosyl α,α-trehalose and about 5% of other saccharide derivatives of α,a-trehalose in total, to give final concentrations of 50 µM of AA-2G and 15.0 mg/ml of saccharides. As a control, test solutions were prepared by dissolving in D-MEM medium either of the above hydrous crystalline α,α-trehalose, α-maltosyl α,α-trehalose, or syrup of saccharide derivatives of α,α-trehalose to give a final concentration of 15.0 mg/ml. The medium admixed with the syrup containing saccharide derivatives of α,α-trehalose was adjusted to give a concentration of 15.0 mg/ml of saccharide derivatives of α,α-trehalose.

### <Method for quantifying collagen-production level in NHDF cells>

The culture of NHDF cells and the quantification of collagen-production level thereof were carried out similarly as in Experiment 1. The results are in Table 3, where the values are expressed with relative values in terms of the collagen production level of NHDF cells, being regarded as 100, when cultured in D-MEM medium containing AA-2G.

**Table 3**

| Kind of saccharide added to culture medium | Addition of AA-2G to medium | Collagen-production level |
|---|---|---|
| None | - | 42 |
| | + | 100 |
| α,α-Trehalose | - | 40 |
| | + | 227 |
| α-Maltosyl α,α-trehalose | - | 39 |
| | + | 182 |
| Syrup containing saccharide derivatives of α,α-trehalose | - | 45 |
| | + | 163 |

| | | |
|---|---|---|
| +: Added to the medium -: Not added to the medium | | |

As evident from Table 3, the collagen-production levels of NHDF cells cultured in the medium free of AA-2G but supplemented with α,α-trehalose, α-maltosyl α,α-trehalose, or the syrup of saccharide derivatives of α,α-trehalose were in the range of 39 to 45, meaning that, when used alone, none of the above-identified saccharides enhanced the action of collagen production as found in AA-2G. While, the NHDF cells gave collagen-production levels of 227, 182 and 163, when cultured in the media with 50 µM of AA-2G and further supplemented with any one of α,α-trehalose, α-maltosyl α,α-trehalose, and the syrup of saccharide derivatives of α,α-trehalose, respectively, revealing that the addition of any of such saccharides enhanced the collagen production by AA-2G.

### <Experiment 4: Influence of α,α-trehalose or a saccharide derivative thereof or a combination use of any of these saccharides and hyaluronic acid and/or chondroitin sulfate on the collagen production by L-ascorbic acids>

Influence of α,α-trehalose or a saccharide derivative thereof, or a combination use of any of these saccharides and hyaluronic acid and/or chondroitin sulfate on the collagen production by L-ascorbic acids was examined as follows.

### <Test solutions>

AA-2G and any one of hydrous crystalline α,α-trehalose, a reagent grade specimen commercialized by Hayashibara Biochemical Laboratories Inc., Okayama, Japan; α-maltosyl α,α-trehalose with a purity of at least 98% commercialized by Hayashibara Biochemical Laboratories Inc., Okayama, Japan; and "HALLODEX", a product name of a syrup of saccharide derivatives of α,α-trehalose were dissolved in D-MEM medium to give final concentrations of 50 µM of AA-2G and 15.0 mg/ml of saccharides. Further, as another test solutions, those, which had been prepared similarly as in the above, were admixed with any one of hyaluronic acid, chondroitin sulfate, and a combination thereof to give respective concentrations of 1 mg/ml, 1 mg/ml, and 0.5 mg/ml of hyaluronic acid and chondroitin sulfate each. The medium supplemented with a syrup containing about 59%, d.s.b., of saccharide derivatives of α,α-trehalose was prepared to give a concentration of 15.0 mg/ml of the saccharide derivatives of α,α-trehalose contained in the syrup.

### <Method for quantifying collagen-production level in NHDF cells>

The culture of NHDF cells and the quantification of collagen-production level thereof were carried out similarly as in Experiment 1. The results are in Table 4 where the values are expressed with relative values in terms of the collagen-production level of NHDF cells, being regarded as 100, when cultured in D-MEM medium containing AA-2G.

**Table 4**

| Kind of saccharide added to medium | Substance added to medium | | | Collagen-production level |
|---|---|---|---|---|
| | AA-2G | Sodium hyaluronate | Chondroitin sulfate | |
| None | - | - | - | 42 |
| | + | - | - | 100 |
| | + | + | - | 98 |
| | + | - | + | 97 |
| | + | + | + | 101 |
| α,α-Trehalose | - | - | - | 42 |
| | + | - | - | 203 |
| | + | + | - | 276 |
| | + | - | + | 256 |
| | + | + | + | 344 |
| α-Maltosyl α,α-trehalose | - | - | - | 39 |
| | + | - | - | 158 |
| | + | + | - | 199 |
| | + | - | + | 188 |
| | + | + | + | 276 |
| Syrup containing saccharide derivatives of α,α-trehalose | - | - | - | 42 |
| | + | - | - | 154 |
| | + | + | - | 192 |
| | + | - | + | 180 |
| | + | + | + | 256 |

| | | | | |
|---|---|---|---|---|
| +: Added to the medium -: Not added to the medium | | | | |

As evident from Table 4, the collagen-production levels of NHDF cells, which had been cultured in the medium free of AA-2G but supplemented with α,α-trehalose, a-maltosyl α,α-trehalose, or the syrup of saccharide derivatives of α,α-trehalose were in the range of 39 to 42, revealing that, when used alone, none of the above-identified saccharides enhanced the action of collagen production as found in AA-2G. While, the NHDF cells gave collagen-production levels of 203, 158 and 154, when cultured in the media with 50 µM of AA-2G and further supplemented with α,α-trehalose, α-maltosyl α,α-trehalose, or the syrup of saccharide derivatives of α,α-trehalose, respectively, revealing that the addition of any of such saccharides enhanced the collagen production by AA-2G similarly as in Experiment 3. In addition, the NHDF cells gave collagen-production levels of 276, 199 and 192, when cultured in respective media with 50 µM of AA-2G and further supplemented with hyaluronic acid and any one of α,α-trehalose, α-maltosyl α,α-trehalose, and the syrup of saccharide derivatives of α,α-trehalose, respectively. While the NHDF cells gave collagen-production levels of 256, 188 and 180, when cultured in respective media with 50 µM of AA-2G and further supplemented with chondroitin sulfate and any one of the above three-types of saccharides. Further, the NHDF cells gave collagen-production levels of 344, 276 and 256, when cultured in respective media with AA-2G and further supplemented with hyaluronic acid and chondroitin sulfate along with any one of the above three types of saccharides and syrup. These results show that hyaluronic acid or chondroitin sulfate more augments the action of α,α-trehalose and saccharide derivatives thereof to enhance the collagen production by L-ascorbic acid. It was confirmed that hyaluronic acid and chondroitin sulfate synergistically augment the action of α,α-trehalose and saccharide derivatives thereof to enhance the collagen production by L-ascorbic acid based on the fact that, when hyaluronic acid and chondroitin sulfate are added in combination, the level of the above-identified action is higher than that attained with only hyaluronic acid or chondroitin sulfate.

### <Experiment 5: Influence of a combination use of α,α-trehalose or saccharide derivatives thereof along with hyaluronic acid and/or chondroitin sulfate>

The following experiment was conducted to examine the influence of α,α-trehalose or saccharide derivatives thereof and hyaluronic acid and/or chondroitin sulfate on the skin. Since there exists a relatively constant amount of L-ascorbic acid in vivo, the collagen production by L-ascorbic acid proceeds constantly. Based on the following basic formulation for lotion and the combination and composition in Table 5, 100 parts by weights of a lotion was prepared by adding to the basic formulation sodium hyaluronate, chondroitin sulfate, hydrous crystalline α,a-trehalose, a cosmetic grade specimen commercialized by Hayashibara Biochemical Laboratories Inc., Okayama, Japan, and "TORNARE", a product name of a syrup having a solid content of about 75% and containing, on a dry solid basis, about 53% by weight of α-maltosyl α,α-trehalose and about 5% by weight of other saccharide derivatives of α,α-trehalose, and an adequate amount of refined water. In Table 5, the respective amounts of the added saccharide are expressed on a dry solid basis. The syrup containing saccharide derivatives of α,α-trehalose was added in an amount of the saccharide derivatives as shown in Table 5.

### <Basic composition ratio for lotion>

| | |
|---|---|
| Glycerine | 5 parts by weight |
| 1,3-Butylene glycol | 6.5 parts by weight |
| Polyoxyethylene sorbitan monolaurate (20 E. O.) | 1.2 parts by weight |
| Ethyl alcohol | 8 parts by weight |
| Lactic acid | 0.05 Part by weight |
| Sodium lactate | 0.1 Part by weight |
| 2-Ethylhexyl 4-methoxycinnamate | 0.1 Part by weight |
| Antiseptic | q.s. |
| Flavor | q.s. |

### <Evaluation of the effect of lotion on skin wrinkles/finely-wrinkles and flabby skin>

Eleven women as volunteers, who had been suffering from skin wrinkles/finely-wrinkles or flabby skin, were allowed to wash their faces with any of the lotions having compositions in Table 5 three times a day for four months and then allowed to evaluate themselves about their face-skin conditions by comparing with those before use of the lotions. It was evaluated based on the following four ranks in terms of changing in skin wrinkles/finely-wrinkles and flabby skin and the number of volunteers for each evaluation, and the results are in Table 5: Remarkably efficacious, meaning that the skin became to have fitness, the depth of wrinkles/finely-wrinkles became slight, and the number of such wrinkles/finely-wrinkles became fewer; efficacious, meaning that the skin became to have fitness, the depth of wrinkles/finely-wrinkles became slight, and the number of such wrinkles/finely-wrinkles became fewer; passable, meaning that the skin became to have fitness but the depth and the number of wrinkles/finely-wrinkles did not change; and inefficacious, meaning that no change was found.

**Table 5**

| Added saccharide | Content of added saccharide (part by weight) | Content of added sodium hyaluronate and/or chondroitin sulfate (part by weight) | | Evaluation (number of volunteers) | | | |
|---|---|---|---|---|---|---|---|
| | | | | A | B | C | D |
| | | Sodium hyaluronate | Chondroitin sulfate | | | | |
| None | 0 | 0 | 0 | 0 | 0 | 1 | 10 |
| | | 0.5 | 0 | 0 | 0 | 2 | 9 |
| | | 0 | 0.5 | 0 | 0 | 1 | 10 |
| | | 0.25 | 0.25 | 0 | 0 | 3 | 8 |
| α,α-Trehalose | 0.05 | 0 | 0 | 0 | 0 | 3 | 8 |
| | 0.1 | 0 | 0 | 0 | 2 | 5 | 4 |
| | 1 | 0 | 0 | 2 | 2 | 6 | 1 |
| | 2 | 0 | 0 | 2 | 4 | 4 | 1 |
| | 10 | 0 | 0 | 3 | 3 | 5 | 0 |
| | 20 | 0 | 0 | 3 | 3 | 5 | 0 |
| | | 0.5 | 0 | 3 | 4 | 3 | 1 |
| | 2 | 0 | 0.5 | 3 | 5 | 3 | 0 |
| | | 0.25 | 0.25 | 6 | 3 | 2 | 0 |
| | 0.05 | 0 | 0 | 0 | 0 | 3 | 8 |
| | 0.1 | 0 | 0 | 0 | 1 | 6 | 4 |
| | 1 | 0 | 0 | 1 | 3 | 6 | 1 |
| Saccharide derivatives of α,α-trehalose | 2 | 0 | 0 | 2 | 3 | 6 | 0 |
| | 10 | 0 | 0 | 2 | 3 | 6 | 0 |
| | 20 | 0 | 0 | 3 | 2 | 6 | 0 |
| | | 0.5 | 0 | 4 | 4 | 2 | 1 |
| | 2 | 0 | 0.5 | 4 | 3 | 4 | 0 |
| | | 0.25 | 0.25 | 6 | 3 | 2 | 0 |

| | | | | | | | |
|---|---|---|---|---|---|---|---|
| Note: The symbols "A", "B", "C" and "D" mean "remarkably efficacious", "efficacious", "slightly efficacious", and "inefficacious", respectively. | | | | | | | |

As evident from Table 5, when the lotions prepared by adding 2 to 20 parts by weight of α,α-trehalose to the lotion with the above-identified basic composition ratio were used, six out of eleven volunteers evaluated them efficacious or more. While, when the lotions prepared by adding 2 to 20 parts by weight of saccharide derivatives of α,α-trehalose to the lotion with the above-identified basic composition ratio were used, five out of eleven volunteers evaluated them efficacious or more. These results revealed that α,α-trehalose and saccharide derivatives of α,α-trehalose have an effect of improving and inhibiting flabby skin, as well as skin wrinkles/finely-wrinkles. Further, it was confirmed that the addition of 0.1 part by weight of α,α-trehalose or saccharide derivatives of α,α-trehalose marked "efficacious" in some volunteers and the addition of one part by weight of any of α,α-trehalose and saccharide derivatives of α,α-trehalose marked "remarkably efficacious" in some volunteers. These results indicate that α,α-trehalose and saccharide derivatives of α,α-trehalose have an effect of improving and inhibiting skin wrinkles/finely-wrinkles. When the lotions prepared by adding sodium hyaluronate and/or chondroitin sulfate and two parts by weight of α,α-trehalose or saccharide derivatives of α,α-trehalose were used, it was found a stronger improving effect on flabby skin and skin wrinkles/finely-wrinkles compared with that exerted with only a sole use of two parts by weight of α,α-trehalose or saccharide derivatives of α,α-trehalose. It was confirmed that, when sodium hyaluronate and chondroitin sulfate were used in combination, the enhancement level of improving effect on flabby skin and skin wrinkles/finely-wrinkles, that is exerted by sodium hyaluronate and/or chondroitin sulfate, is higher than that exerted by their single use. None of the volunteers evaluated the lotions, which had the above-identified basic composition ratio with or without sodium hyaluronate and/or chondroitin sulfate in the amounts as shown in Table 5, as "efficacious" or "slightly efficacious".

### <Experiment 6: Influence of a combination use of α,α-trehalose or saccharide derivatives thereof, sodium hyaluronate and/or chondroitin sulfate, and adenosine monophosphate and/or coenzyme Q10 on the skin>

The influence of a combination use of α,α-trehalose or saccharide derivatives thereof, sodium hyaluronate and/or chondroitin sulfate, and adenosine monophosphate (may be abbreviated as "AMP", hereinafter) and/or coenzyme Q10 (may be abbreviated as "CoQ10", hereinafter) on the skin was examined by the following experiment. One hundred parts by weight of respective lotions were prepared by adding to fresh preparations of the same lotion with the basic composition ratio as used in Experiment 5 sodium hyaluronate, chondroitin sulfate, hydrous crystalline α,α-trehalose, a cosmetic grade specimen commercialized by Hayashibara Biochemical Laboratories Inc., Okayama, Japan, and "TORNARE", a product name of a syrup having saccharide derivatives of α,α-trehalose commercialized by Hayashibara Biochemical Laboratories Inc., Okayama, Japan, AMP, and CoQ10 according to the combinations and compositions as in Table 6 below. The amounts of added saccharides are represented based on a dry solid basis. The syrup containing saccharide derivatives of α,α-trehalose was added to each lotion to give respective composition ratios as in Table 6. Any of the lotions was allowed to be used every day by 11 females, who had been suffering from flabby skin or skin wrinkles/finely-wrinkles, three times a day for three months, and the results were evaluated on flabby skin or skin wrinkles/finely-wrinkles by the method similarly as in Experiment 5. The results are in Table 6.

**Table 6**

| Added saccharide and its content | Content of added sodium hyaluronate, chondroitin sulfate, AMP and/or CoQ10 (part by weight) | | | | Evaluation (number of volunteers) | | | |
|---|---|---|---|---|---|---|---|---|
| | Sodium hyaluronate | Chondroitin sulfate | AMP | CoQ10 | A | B | C | D |
| α,α-Trehalose (one part by weight) | 0 | 0 | 0 | 0 | 1 | 2 | 6 | 2 |
| | 0 | 0 | 0.2 | 0 | 2 | 2 | 6 | 1 |
| | 0 | 0 | 0 | 0.2 | 1 | 3 | 6 | 1 |
| | 0 | 0 | 0.1 | 0.1 | 2 | 3 | 5 | 1 |
| | 0.1 | 0.1 | 0 | 0 | 2 | 4 | 4 | 1 |
| | 0.1 | 0.1 | 0.2 | 0 | 3 | 4 | 4 | 0 |
| | 0.1 | 0.1 | 0 | 0.2 | 4 | 3 | 4 | 0 |
| | 0.1 | 0.1 | 0.1 | 0.1 | 5 | 5 | 1 | 0 |
| Saccharide derivatives of α,α-trehalose (one part by weight) | 0 | 0 | 0 | 0 | 1 | 1 | 6 | 3 |
| | 0 | 0 | 0.2 | 0 | 1 | 2 | 6 | 2 |
| | 0 | 0 | 0 | 0.2 | 1 | 2 | 5 | 3 |
| | 0 | 0 | 0.1 | 0.1 | 2 | 2 | 5 | 2 |
| | 0.1 | 0.1 | 0 | 0 | 2 | 3 | 5 | 1 |
| | 0.1 | 0.1 | 0.2 | 0 | 3 | 3 | 5 | 0 |
| | 0.1 | 0.1 | 0 | 0.2 | 3 | 4 | 4 | 0 |
| | 0.1 | 0.1 | 0.1 | 0.1 | 5 | 4 | 2 | 0 |

| | | | | | | | | |
|---|---|---|---|---|---|---|---|---|
| Note: The symbols "A", "B", "C" and "D" mean "remarkably efficacious", "efficacious", "slightly efficacious", and "inefficacious", respectively. | | | | | | | | |

As evident from Table 6, when the lotions, which had been prepared by adding α,α-trehalose or saccharide derivatives thereof along with AMP and/or CoQ10 to the lotion with the above-identified basic composition ratio, were used, there was found a stronger improving effect on flabby skin and skin wrinkles/finely-wrinkles compared with that attained when α,α-trehalose or saccharide derivatives thereof was used alone. It was confirmed that, when AMP and CoQ10 are incorporated in combination into the lotions, the enhancement effect of AMP and/or CoQ10 on the improvement of flabby skin and skin wrinkles/finely-wrinkles exerted by α,α-trehalose or saccharide derivatives thereof is stronger than the level attained when AMP or CoQ10 is incorporated alone. A stronger effect of improving flabby skin and skin wrinkles/finely-wrinkles was observed when the solution, which had been prepared by adding α,α-trehalose or saccharide derivatives thereof along with 0.1 part by weight of hyaluronic acid, 0.1 part by weight of chondroitin sulfate, and further AMP and/or CoQ10. The above effect was higher than that attained with the lotions prepared by adding α,α-trehalose or saccharide derivatives thereof along with 0.1 part by weight of hyaluronic acid, and 0.1 part by weight of chondroitin sulfate. It was confirmed that the enhancement effect of AMP and/or CoQ10 on the effect of improving flabby skin and skin wrinkles/finely-wrinkles exerted by the lotions containing α,α-trehalose or saccharide derivatives thereof along with sodium hyaluronate and chondroitin sulfate is stronger than that attained with the lotions incorporated with AMP or CoQ10 alone.

These experimental results show the facts that α,α-trehalose and saccharide derivatives thereof have a distinct action of enhancing the collagen production by L-ascorbic acids and the compositions containing α,α-trehalose or saccharide derivatives thereof can be used as an agent for enhancing the collagen production by L-ascorbic acids, improving/inhibiting flabby skin, and for improving/inhibiting wrinkles/finely-wrinkles. The fact that sodium hyaluronate and/or chondroitin sulfate and AMP and/or CoQ10 more augment the action of α,α-trehalose and saccharide derivatives thereof to enhance the collagen production by AA-2G indicates that compositions, prepared by adding one or more ingredients selected from sodium hyaluronate, chondroitin sulfate, AMP, and CoQ10 to a composition containing α,α-trehalose or saccharide derivatives thereof, can be more advantageously used as an agent for enhancing the collagen production by L-ascorbic acid, improving/inhibiting flabby skin, and for improving/inhibiting wrinkles/finely-wrinkles than those containing α,α-trehalose or saccharide derivatives thereof but with no addition of the above ingredients.

The following Examples explain the present invention in more detail but they do not limit the scope of the present invention.

### Example 1

### Collagen-productin enhancer

The following ingredients were mixed to homogeneity according to the composition ratio as indicated below to obtain a collagen-production enhancer.

| | |
|---|---|
| "TREHA", a product name of hydrous crystalline α,α-trehalose commercialized by Hayashibara Shoji, Inc. Okayama, Japan | 49 parts by weight |
| "AA2G", a product name of L-ascorbic acid 2-glucoside commercialized by Hayashibara Biochemical Laboratories Inc., Okayama, Japan | 1 Part by weight |

The product is a readily usable, efficacious collagen-production enhancer that exhibits an action of enhancing collagen-production, imparts moisture to the skin, and exerts an effect on preventing the skin aging. Since the product has an adequate sour taste to impart satisfactory taste, it can be orally taken intact as a supplemental health food for enhancing collagen-production with an index for use in collagen-production enhancement, or taken arbitrarily after dissolving in water or other beverages. The product can be arbitrarily added to foods for special dietary uses, health-promoting foods, cosmetics, quasi-drugs, pharmaceuticals, feeds, baits for fish, pet foods, other daily goods, etc., to impart them a collagen-production action.

One gram aliquots of the product was tabletted into a confectionery in the form of a tablet. The product can be commercialized with an index for use in collagen production.

### Example 2

### <Supplemental health food>

The following ingredients were mixed to homogeneity according to the following composition ratio to obtain a supplemental health food for enhancing collagen-production. The product was diluted with a fresh preparation of the same D-MEM medium as used in the above Experiment 1 and subjected to measurement of its collagen-production-enhancing activity, confirming that it exerted the desired activity.

| | |
|---|---|
| L-Ascorbic acid | 5 parts by weight |
| *Ganoderma lucidum,* commercialized by Hayashibara Biochemical Laboratories Inc., Okayama, Japan | 1.5 parts by weight |
| Indigo, commercialized by Hayashibara Biochemical Laboratories Inc., Okayama, Japan | 1.5 parts by weight |
| "HALLODEX", a product name of a syrup of saccharide derivatives of α,α-trehalose, commercialized by Hayashibara Shoji, Inc. Okayama, Japan | 25 parts by weight |
| "TREHA", a product name of hydrous crystalline α,α-trehalose, commercialized by Hayashibara Shoji, Inc. Okayama, Japan | 10 parts by weight |

The product can be used as a supplemental health food suitable for collagen-production enhancement, which can be provided with an index for use in such purpose. The product is preferable as a daily usable supplemental health food which has a satisfactory effect on normalization and anti-aging of skin tissues, as well as having a satisfactory taste due to its mild sweetness and adequate sour taste. Also the product can be arbitrarily used as a composition administered orally or intubationally to humans and animals such as livestocks and pet animals, or used intact or after mixed with feeds for raising animals such as fish, shrimp/lobster, and crab.

To the supplemental health food for collagen-production enhancement was added sucrose fatty acid ester to give a concentration of one percent by weight, and the resulting mixture was formed by a tabletting machine into tablets, about 300 mg each. The product thus obtained is a supplemental health food for collagen-production enhancement, which exerts a successive collagen-production-enhancing-action, has a relatively long shelf-life, convenient portability, and readily oral-swallowability, and which exerts a desired distinct effect. The product can be also used as a supplemental health food usable for enhancing collagen-production, which can be provided with an index for use in such purpose.

### Example 3

### <Supplemental health food>

The following ingredients were mixed to homogeneity according to the following composition ratio to obtain a powdery supplemental health food for enhancing collagen-production. After preparation, it was confirmed that the product stably exerts a collagen-production enhancement.

| | |
|---|---|
| "TREHA", a product name of hydrous crystalline α,α-trehalose, commercialized by Hayashibara Shoji, Inc. Okayama, Japan | 5.5 parts by weight |
| "ASCOFRESH", a product of L-ascorbic acid 2-glucoside, commercialized by Hayashibara Shoji, Inc. Okayama, Japan | 1.5 parts by weight |
| Sodium chondroitin sulfate | 1 part by weight |
| Chitosan | 1 part by weight |
| "αG RUTIN", a saccharide-transferred rutin | 1 part by weight |
| Adenosine monophosphate | 0.1 part by weight |
| Coenzyme Q10 | 0.05 part by weight |

The product can be used as a supplemental health food usable for enhancing collagen-production, which can be provided with an index for use in such purpose. Since the product has a satisfactory effect on normalization and anti-aging of skin tissues and has a satisfactory taste due to its mild sweetness and adequate sour taste, it is preferable as a daily usable supplemental health food.

### Example 4

### <Supplemental health beverage>

| | |
|---|---|
| Powdered maltitol | 500 parts by weight |
| Collagen-production enhancer prepared by the method in Example 1 | 100 parts by weight |
| Powdered egg yolk | 190 parts by weight |
| Skim milk | 200 parts by weight |
| Sodium chloride | 4.4 parts by weight |
| Potassium chloride | 1.85 parts by weight |
| Magnesium sulfate | 4 parts by weight |
| Chondroitin sulfate | 0.1 part by weight |
| Thiamine | 0.01 part by weight |
| Vitamin E acetate | 0.6 part by weight |
| Nicotinic acid amide | 0.04 part by weight |
| "αG HESPERIDIN PS", a product name of saccharide-transferred hesperidin, commercialized by Toyo Sugar Refining Co., Ltd., Tokyo, Japan | 0.02 part by weight |

A composition having the above composition ratio was prepared. Twenty-five parts by weight of the composition was dispersed and dissolved to homogeneity in 150 parts by weight of refined water, and 200 g aliquots of the resulting solution were respectively placed and sealed in brown colored glass-vials.

The product is a supplemental health beverage usable for enhancing collagen-production, which can be provided with an index for use in such purpose. Since the product continuously exerts collagen-production enhancement, has supplemental nutritional sources, and has a delicious beverage taste with reduced unfavorable taste and smell inherent to the above ingredients, it can be arbitrarily used for beauty and health. The product can be arbitrarily used as a composition to be administered orally or intubationally to humans and animals such as livestocks and pet animals. Since the product contains saccharide-transferred hesperidin, it can be arbitrarily called for its use to reduce the level of blood lipids such as lipids and cholesterol in blood.

### Example 5

### <Supplemental health food>

| | |
|---|---|
| Powdered maltitol | 25 parts by weight |
| "TREHA", a product name of hydrous crystalline α,α-trehalose, commercialized by Hayashibara Shoji, Inc. Okayama, Japan | 67 parts by weight |
| Gloss-imparting agent | 3 parts by weight |
| Glucosamine | 2.5 parts by weight |
| Sodium chondroitin sulfate | 1.5 parts by weight |
| Hyaluronic acid | 1 part by weight |

Based on the above composition ratio, the ingredients were mixed to homogeneity, and 0.5 g aliquots of which were respectively tabletted in conventional manner to obtain tablets.

The product is a supplemental health food usable for enhancing collagen-production, which can be provided with an index for use in such purpose. Since the product, which continuously exerts a collagen-production enhancement and contains glucosamine, chondroitin sulfate, and hyaluronic acid, it has a particularly advantageous action of strengthening bone tissues and articulations. The product is a tasteful health food with reduced unfavorable taste and smell inherent to glucosamine, chondroitin sulfate, and hyaluronic acid, and it can be arbitrarily used for beauty and health. The product can be arbitrarily used as a composition administered orally or intubationally to humans and animals such as livestocks and pet animals.

### Example 6

### <Supplemental health food>

| | |
|---|---|
| "SUNMALT S", a product name of a purified maltose, commercialized by Hayashibara Shoji, Inc., Okayama, Japan | 25 parts by weight |
| "TREHA", a product name of hydrous crystalline α,α-trehalose, commercialized by Hayashibara Shoji, Inc. Okayama, Japan | 54.2 parts by weight |
| Gloss-imparting agent | 5 parts by weight |
| Oyster shell calcium | 2 parts by weight |
| Hyaluronic acid | 0.2 part by weight |
| "ASCOFRESH", a product of L-ascorbic acid 2-glucoside, commercialized by Hayashibara Shoji, Inc. Okayama, Japan | 10 parts by weight |
| "αG RUTIN P", a product name of saccharide-transferred rutin, commercialized by Toyo Sugar Refining Co., Ltd., Tokyo, Japan | 0.5 part by weight |
| Sour agent (organic acids) | q.s. |
| Lemon and lime flavor | q.s. |

The above ingredients were volumed up to give a total amount of 100 parts by weight and mixed to homogeneity by stirring, and 0.5 g aliquots of the resulting mixture were respectively tabletted in conventional manner to obtain tablets.

The product is a supplemental health food usable for enhancing collagen-production, which can be provided with an index for use in such purpose. Since the product, which continuously exerts a collagen-production enhancement, contains hyaluronic acid, it has an improved action of improving flabby skin, strengthening bone tissues and articulations, and improving arthralgia. The product is a tasteful supplemental health food and it can be also arbitrarily used for beauty and health. The product can be further used as a composition administered orally or intubationally to humans and animals such as livestocks and pet animals.

### Example 7

### <Ice cream>

| | |
|---|---|
| Raw cream (fat and oil contents of about 46%) | 18 parts by weight |
| Skim milk | 7 parts by weight |
| Whole milk | 51 parts by weight |
| Sugar | 13 parts by weight |
| Pullulan | 2 parts by weight |
| Guar gum | 1 part by weight |

The composition with the above composition ratio was dissolved, sterilized by keeping at 70° C for 30 min, emulsified and dispersed by a homogenizer, instantly cooled to 3 to 4°C, and admixed with five parts by weight of "HALLODEX", a product name of a syrup of saccharide derivatives of α,a-trehalose, commercialized by Hayashibara Shoji, Inc. Okayama, Japan. The mixture was allowed to stand overnight and freezed in a freezer to obtain an ice cream.

The product can be used to enhance collagen-production with an index for use in such purpose as an ice cream capable of imparting moisture to the skin and preventing skin aging.

### Example 8

### <Fruit jelly>

| | |
|---|---|
| Sugar | 14 parts by weight |
| "TREHA", a product name of hydrous crystalline α,α-trehalose, commercialized by Hayashibara Shoji, Inc. Okayama, Japan | 4 parts by weight |
| Gelatin | 2.5 parts by weight |
| Grape fruit juice | 34 parts by weight |
| Water | 45.5 parts by weight |

Based on the above composition ratio, sugar, α,α-trehalose, gelatin, and water were admixed, and the mixture was dissolved by heating at 95°C, and then admixed with grape the fruit juice, and further heated at 80°C for 30 min to effect sterilization, followed by cooling the mixture to obtain a fruit jelly.

The product can be used for collagen-production enhancement with an index for use in such purpose. The product has an adequate sweetness and smooth texture, prevents skin aging, and exerts an effect of maintaining and promoting beauty and health.

### Example 9

### <Tea beverage>

Fifty grams of a green tea blended for liquid beverage was extracted with 1,500 g of ion-exchanged water at 65°C for five minutes, and the extract was filtered with a paper filter to remove tea leaves to obtain 1,320 g of a green tea extract (pH 6.0). The extract was diluted by about 5-times with ion-exchanged water to adjust its concentration suitable for drinking. The dilute was admixed with L-ascorbic acid in an amount sufficient to give a concentration of 300 ppm and adjusted to give a pH 6.0 with sodium hydrogen carbonate to obtain a mixture solution. To the mixture solution were added "TREHA", a product name of hydrous crystalline α,α-trehalose, commercialized by Hayashibara Shoji, Inc. Okayama, Japan, and a saccharide-transferred hesperidin with a purity of at least 95%, commercialized by Hayashibara Co., Ltd., Okayama, Japan, to give respective concentrations of 0.75% and 1%. Three hundred grams aliquots of the resulting solution were respectively injected and sealed in thermostable transparent glass vessels and subjected to retort sterilization at 121° C for seven minutes to obtain a green tea beverage.

The product can be used for enhancing collagen production with an index for use in such purpose, and it is a tea beverage effective in preventing skin aging, as well as maintaining and promoting beauty and health. Since the product contains saccharide-transferred hesperidin, it can be arbitrarily called for its use to reduce the level of blood lipids such as neutral fat and cholesterol in blood.

### Example 10

### <External dermal cream>

The following composition A was admixed with the composition B in usual manner, and the mixture was cooled to 30° C or lower, adjusted to a slightly alkaline pH with potassium hydroxide, and emulsified by a homogenizer to obtain an external dermal cream.

| <Composition A> | |
|---|---|
| Polyoxyethylene glyceryl monostearate | 2 parts by weight |
| Glyceryl monostearate, self-emulsifying | 5 parts by weight |
| Eicosanyl behenate | 1 part by weight |
| Liquid petrolatum | 1.9 parts by weight |
| Trimethylolpropane trioctanoate | 10 parts by weight |

| <Composition B> | |
|---|---|
| 1,3-Butylene glycol | 4 parts by weight |
| α,α-Trehalose, a reagent grade specimen, commercialized by Hayashibara Biochemical Laboratories Inc., Okayama Japan | 3 parts by weight |
| "TORNARE", a product name of a syrup containing saccharide derivatives of α,a-trehalose, commercialized by Hayashibara Biochemical Laboratories Inc., Okayama, Japan | 1 part by weight |
| Sodium lactate solution | 10 parts by weight |
| "AA2G", a product name of L-ascorbic acid 2-glucoside, commercialized by Hayashibara Biochemical Laboratories Inc., Okayama, Japan | 2 part by weight |
| Eelgrass *(Zostera marina)* extract | 0.5 part by weight |
| Soybean extract | 0.5 part by weight |
| *Aloe vera* extract | 0.5 part by weight |
| Methyl parahydroxybenzoate | 0.1 part by weight |
| Sodium chondroitin sulfate | 0.1 part by weight |
| Glycyrrhiza extract | 0.5 part by weight |
| Refined water | 62.4 parts by weight |

The external dermal cream can be optionally made into a slightly alkaline cream by the addition of potassium hydroxide.

Since the product enhances and maintains collagen production in the skin, it improves flabby skin, exerts an effect on wrinkles/finely-wrinkles, and satisfactorily prevents skin aging. The product has an improved moisture-retaining ability and it can be used as a basic cosmetic.

### Example 11

### <Lotion>

Based on the following composition ratio, a lotion was prepared in usual manner.

| | |
|---|---|
| "TORNARE", a product name of a syrup containing saccharide derivatives of α,α-trehalose, commercialized by Hayashibara Biochemical Laboratories Inc., Okayama, Japan | 10 parts by weight |
| "AA2G", a product name of L-ascorbic acid 2-glucoside, commercialized by Hayashibara Biochemical Laboratories Inc., Okayama, Japan | 2 parts by weight |
| Dipotassium glycyrrhizinate | 0.05 part by weight |
| Ethanol | 10 parts by weight |
| 1,3-Butylene glycol | 5 parts by weight |
| "α-GLUCOSYL HESPERIDIN", a product name of a saccharide-transferred hesperidin, commercialized by Hayashibara Biochemical Laboratories Inc., Okayama, Japan | 1 part by weight |
| Extract of blue-green algae extracted with water | 1 part by weight |
| Adenosine monophosphate | 0.5 part by weight |
| Coenzyme Q10 | 0.5 part by weight |
| Sodium hyaluronate | 0.1 part by weight |
| KANKOSO-201 | 0.005 part by weight |
| Polyoxyethylene(60)hydrogenated castor oil | 1 part by weight |
| Water | q. s. sufficient to give a total amount of 100 parts by weight |

Since the product enhances and maintains collagen production in the skin, it maintains moisture in the skin, improves flabby skin, exerts an effect on wrinkles/finely-wrinkles, and satisfactorily prevents skin aging. Since the extract of blue-green algae and dipotassium glycyrrhizinate exert an improved anti-inflammatory and skin-whitening effect, the product is useful as a basic or skin-whitening cosmetic.

### Example 12

### <Lotion>

The following composition A was dissolved in refined water into an aqueous solution. The composition B was weighed, dissolved by heating, and admixed with the composition C to form a gel. To the gel was added the above aqueous solution, and the mixture was stirred until homogeneity in usual manner to obtain a lotion.

| <Composition A> | |
|---|---|
| 1,3-Butylene glycol | 5 parts by weight |
| "TORNARE", a product name of a syrup containing saccharide derivatives of α,α-trehalose, commercialized by Hayashibara Biochemical Laboratories Inc., Okayama, Japan | 3 parts by weight |
| Sclerotium gum solution (1% β-glucan solution) | 3 parts by weight |
| Citric acid | 0.01 part by weight |
| Sodium citrate | 0.04 part by weight |
| Propyl parahydroxybenzoate | 0.2 part by weight |
| Refined water | 85.65 parts by weight |

| <Composition B> | |
|---|---|
| Phytosteryl hydroxystearate | 0.04 part by weight |
| Glyceryl tri-2-ethylhexanoate | 0.24 part by weight |
| Isostearyl alcohol | 0.12 part by weight |
| Squalane | 0.18 part by weight |
| Polyoxyethylene hydrogenated castor oil | 0.69 part by weight |
| Polyglyceryl diisostearate | 0.46 part by weight |
| Propyl parahydroxybenzoate | 0.1 part by weight |
| Glycerine | 1.13 parts by weight |

| <Compound C> | |
|---|---|
| Refined water | 0.14 part by weight |

Since the product is a microemulsion with a small amount of stably incorporated oily ingredients, which enhances and maintains collagen production in the skin, it keeps moisture in the skin, improves flabby skin, exerts an effect on wrinkles/finely-wrinkles, and satisfactorily prevents skin aging. The product has an improved feeling of use as an external dermal agent containing saccharide derivatives of α,α-trehalose, imparts a durable moistened feeling, and has a satisfactory humectancy because it contains scleroglucan, a water-soluble polysaccharide.

### Example 13

### <Essence>

The following composition A was weighed and dissolved by warming up. To the mixture was added the composition B, which had been weighed and dissolved by warming up, and the resulting mixture was in usual manner cooled to ambient temperature while stirring to obtain an essence.

| <Composition A> | |
|---|---|
| 25% of a soybean lysophospholipid solution | 1 part by weight |
| "TORNARE", a product name of a syrup containing saccharide derivatives of α,α-trehalose, commercialized by Hayashibara Biochemical Laboratories Inc., Okayama, Japan | 4 parts by weight |
| 1,3-Butylene glycol | 5 Parts by weight |
| 1% scleroglucan solution | 10 parts by weight |
| 2% quince seed extract | 10 parts by weight |
| Methyl parahydroxybenzoate | 0.2 part by weight |
| Refined water | 54.9 parts by weight |

| <Composition B> | |
|---|---|
| Sorbitan monostearate | 0.5 part by weight |
| Glyceryl monostearate, self-emulsifying | 1 part by weight |
| Cetanol | 1 part by weight |
| Phytosteryl hydroxystearate | 0.3 part by weight |
| Di(phytostearyl-2-octyldodecyl) N-lauroyl-L-glutamate | 0.5 part by weight |
| Squalane | 6 parts by weight |
| 2-Ethylhexyl palmitate | 2 parts by weight |
| Glyceryl tri-2-ethylhexanoate | 2 parts by weight |
| Propyl parahydroxybenzoate | 0.1 part by weight |
| Methyl polysiloxane | 0.5 part by weight |

Since the product has an action of enhancing and maintaining collagen production in the skin, it keeps moisture in the skin, improves flabby skin, exerts an effect on wrinkles/finely-wrinkles, and satisfactorily prevents skin aging. Since the product contains scleroglucan and quince seed extract, it has an improved feeling of use as an external dermal agent containing saccharide derivatives of α,a-trehalose, imparts a durable moistened feeling, and has a satisfactory humectancy.

### Example 14

### <D-Phase preparation>

The following composition A was dissolved by heating and homogeneously admixed with the composition B, which had been dissolved by heating, and then stirred and mixed in usual manner while gradually adding thereto refined water and cooled to ambient temperature to obtain D-phase preparation.

| <Composition A> | |
|---|---|
| Polyglyceryl stearate-10 | 1.75 parts by weight |
| Polyglyceryl myristate-10 | 5.2 parts by weight |
| "TORNARE" a product name of a saccharide containing saccharide derivatives of α,α-trehalose, commercialized by Hayashibara Biochemical Laboratories Inc., Okayama, Japan | 16.4 parts by weight |
| Anhydrous crystalline α,α-trehalose, a cosmetic specimen, commercialized by Hayashibara Biochemical Laboratories Inc., Okayama, Japan | 1 part by weight |
| Glycerine | 8.7 parts by weight |
| Methylparaben | 0.13 part by weight |

| <Composition B> | |
|---|---|
| Trioctanoin (triethylhexanoin) | 52.22 parts by weight |
| Tocopherol | 0.05 part by weight |
| Propylparaben | 0.05 part by weight |
| Water | an amount sufficient to give a total amount of 100 parts by weight |

Since the product enhances and maintains collagen production in the skin, it keeps moisture in the skin, improves flabby skin, exerts an effect on wrinkles/finely-wrinkles, and satisfactorily prevents skin aging. Because of this, it can be advantageously used as a basic cosmetic. Also the product has a reduced surface tension due to saccharide derivatives of α,α-trehalose, it has properties of being formed into a more fine, stable emulsified drop compared with conventional D-phase preparations prepared with polyhydric alcohol. Because of this, the product can be used by being incorporated into cleansing gels, lotions, bath salts, etc.

### Example 15

### <Pack agent in the form of a jelly, peel-off-type>

Based on the following composition ratio, a pack agent was prepared in usual manner.

| | |
|---|---|
| Poly(vinyl alcohol) | 7 parts by weight |
| Pullulan | 7 parts by weight |
| 1,3-Butyleneglycol | 3 parts by weight |
| Glycerol | 15 parts by weight |
| "TORNARE", a product name of a saccharide containing saccharide derivatives of α,α-trehalose, commercialized by Hayashibara Biochemical Laboratories Inc., Okayama, Japan | 12 parts by weight |
| Ethanol | 8 parts by weight |
| "AA2G", a product name of L-ascorbic acid 2-glucoside, commercialized by Hayashibara Biochemical Laboratories Inc., Okayama, Japan | 1 part by weight |
| POE Oleyl alcohol | 0.5 part by weight |
| Deep ocean sea | 0.5 part by weight |
| Flavor | q.s. |
| Buffer | q.s. |
| Refined water | 15 parts by weight |

Since the product enhances and maintains collagen production in the skin, it keeps moisture in the skin, improves flabby skin, exerts an effect on wrinkles/finely-wrinkles, and exerts a satisfactory effect on preventing skin aging and retaining moisture in the skin. The product has an improved feeling of use, readiness of being wiped or detached from the skin after partial or complete drying, and has a satisfactory cleansing effect on skin surfaces and pores.

### Example 16

### <Dentifrice>

Based on the following composition ratio, a dentifrice was prepared in usual manner. Since the product contains α,α-trehalose and saccharide derivatives thereof, it enhances collagen production in alveolar arch cells and inhibits reduction of alveolar arch due to aging and gingivitis. Also the product foams easily, imparts a satisfactory feeling of use after washing, and removes rhyparia clearly. The product has improved anticariogenicity due to α,α-trehalose and saccharide derivatives thereof and effectively inhibits the formation of adhesive insoluble glucans, formed from sucrose by the action of glucanotransferase derived from microorganisms of the species *Streptococcus mutans,* a dental caries-inducing microorganism.

| | |
|---|---|
| β-Glycyrrhizinic acid | 0.4 part by weight |
| Cetylpyridinium chloride | 0.2 part by weight |
| Tocopherol acetate | 0.1 part by weight |
| Anhydrous crystalline α,α-trehalose, a cosmetic specimen, commercialized by Hayashibara Biochemical Laboratories Inc., Okayama, Japan | 6 parts by weight |
| Concentrated glycerine | 10 parts by weight |
| "TORNARE", a product name of a saccharide containing saccharide derivatives of α,α-trehalose, commercialized by Hayashibara Biochemical Laboratories Inc., Okayama, Japan | 9 parts by weight |
| Refined water | 23.5 parts by weight |
| Ethanol | 3 parts by weight |
| Propolis extract | 1 part by weight |
| Extract of blue-green algae extracted with water | 1 part by weight |
| L-Ascorbic acid 2-glucoside, a reagent grade specimen, commercialized by Hayashibara Biochemical Laboratories Inc., Okayama, Japan | 2 parts by weight |
| Sodium hyaluronate | 0.1 part by weight |
| Carrageenan | 0.3 part by weight |
| Sodium carboxymethyl cellulose | 0.3 part by weight |
| Sodium secondary phosphate | 20 parts by weight |
| Calcium phosphate hydroxide | 3.5 parts by weight |
| Calcium carbonate | 1 part by weight |
| Titanium oxide | 0.4 part by weight |
| Silicic acid anhydride | 0.1 part by weight |
| Sodium lauryl sulfate | 1 part by weight |
| Sodium N-lauroyl sarcosinate | 0.5 part by weight |

### Example 17

### <Peritoneal dialysate>

Six grams of a syrup containing α-maltosyl α,α-trehalose with a purity of 98.5% and a solid content of 75%, commercialized by Hayashibara Biochemical Laboratories Inc., Okayama, Japan; 2.1 grams of L-ascorbic acid 2-glucoside with a purity of 99.5%, commercialized by Hayashibara Biochemical Laboratories Inc., Okayama, Japan; 0.567 g of sodium chloride; 3.92 g of sodium lactate; 0.294 g of calcium chloride; and 0.103 g of magnesium chloride were dissolved in distilled water, and the resulting solution was adjusted to a pH of about 7.5 with 0.294 g of sodium hydroxide and volumed up to give a total amount of 1,000 ml with water. The solution thus obtained was filtered with a 0.2 µm membrane filter, and the filtrate was injected into a poly(vinyl chloride) resin (PVC) bag and autoclaved at 121°C for 20 min. The produced was prepared by using materials, which had been removed microorganisms and pyrogens. The product can be prepared by using buffers such as sodium bicarbonate in place of the above sodium lactate.

The product is a peritoneal dialysate, which enhances collagen production in peritoneal organs and walls and strengthens peritoneal tissues and organs. The product has a satisfactory storage stability and improved biocompatibility and it can be used as a dialysate for hemodialysis, wash for wounds and organs, peritoneal and pleural perfusate, preserving solutions, etc.

### Industrial Applicability

As explained above, the present invention was made based on a completely novel self-finding that a collagen-production enhancer containing α,α-trehalose and/or saccharide derivatives thereof exhibits a distinct action of effectively enhancing the collagen production by L-ascorbic acids when administered to humans and animals. The collagen-production enhancer has no fear of inducing serious side effect and it can be easily and comfortably used for humans and animals to prevent skin aging and to maintain/promote beauty and health. Also the collagen-production enhancer with such properties can be used with other daily goods as compositions such as foods, beverages, foods for special dietary uses, health-promoting foods, cosmetics, quasi-drugs, pharmaceuticals, feeds, baits for fish, pet foods, other daily goods, etc. The present invention with such outstanding functions and effects is a significant invention that greatly contributes to this art.

## Claims

1. A collagen-production enhancer, which comprises as an effective ingredient α,α-trehalose and/or a saccharide derivative thereof.

2. The collagen-production enhancer of claim 1, which further contains L-ascorbic acid and/or the like.

3. The collagen-production enhancer of claim 1 or 2, which further contains glucosamine and/or a mucopolysaccharide.

4. The collagen-production enhancer of claim 3, which further contains one or more members selected from the group consisting of adenosine, derivatives thereof, and salts of said adenosine and said derivatives in the form of a mono-, di-, or tri-phosphate; and/or coenzyme Q10 (CoQ10).

5. The collagen-production enhancer of any one of claims 2 to 4, wherein said L-ascorbic acid and/or the like is L-ascorbic acid 2-glucoside.

6. The collagen-production enhancer of any one of claims 1 to 5, which contains said α,α-trehalose and/or said saccharide derivative thereof in a total amount of at least one percent by weight, on a dry solid basis.

7. A composition which is used for enhancing collagen-production, said composition comprising the collagen-production enhancer as claimed in any one of claims 1 to 6 and having an index of enhancing collagen production.

8. The collagen-production enhancer of any one of claims 1 to 6, which is used as an agent for inhibiting and/or improving the formation of flabby skin, wrinkles, or finely wrinkles.
